# EUROPEAN PATENT APPLICATION

(11) **EP 0 759 468 A1**
(43) Date of publication of application: **26.02.1997**
(21) Application number: 95401879.2
(22) Date of filing: 10.08.1995
(51) Int. Cl.: C12N 15/24, A61K 38/20, C07K 14/54, C07K 16/24, C12N 5/10

(54) **Feline interleukin-4**

(71) Applicant: LABORATOIRES VIRBAC, 06516 Carros (FR)
(72) Inventor: Schijns, Virgil E.C.J., Virology Division, NL-3584 CL Utrecht (NL); Horzinek, Marian C., Virology Division, NL-3584 CL Utrecht (NL)
(74) Representative: Desaix, Anne

(57) **Abstract**

Feline Interleukin-4 is produced in useful quantities using recombinant DNA techniques. Feline Interleukin-4 as well as novel mutant forms of feline Interleukin-4 are utilized for therapeutical treatment of a variety of diseases and as immunological adjuvants. Feline Interleukin-4 nucleotide probes, primers and antibodies are also disclosed.

SEQUENCE LISTING

(1) GENERAL INFORMATION:

(i) APPLICANT:
(A) NAME: LABORATOIRES VIRBAC
(B) STREET: 1ere Avenue - 2065m - L.I.D.
(C) CITY: CARROS
(E) COUNTRY: FRANCE
(F) POSTAL CODE (ZIP): 06516

(ii) TITLE OF INVENTION: FELINE INTERLEUKIN-4

(iii) NUMBER OF SEQUENCES: 9

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 95401879.2

(2) INFORMATION FOR SEQ ID NO: 1:

(i) SEQUENCE CHARACTERISTICS:
(A) LENGTH: 519 base pairs
(B) TYPE: nucleic acid
(C) STRANDEDNESS: double
(D) TOPOLOGY: linear

(ii) MOLECULE TYPE: cDNA

(iii) HYPOTHETICAL: NO

(iv) ANTI-SENSE: NO

(ix) FEATURE:
(A) NAME/KEY: CDS
(B) LOCATION:4..402

(xi) SEQUENCE DESCRIPTION: SEQ ID NO: 1:

TTA ATG GGT CTC ACC TAC CAA CTG ATT CCA GCT CTG GTC TGC TTA CTA 48
Met Gly Leu Thr Tyr Gln Leu Ile Pro Ala Leu Val Cys Leu Leu

1                   5                   10                  15

GCA TTT ACC AGC ACC TTC GTC CAC GGC CAG AAC TTC AAT AAT ACG TTG          96
Ala Phe Thr Ser Thr Phe Val His Gly Gln Asn Phe Asn Asn Thr Leu
                20                  25                  30

AAA GAG ATC ATC AAA ACG TTG AAC ATC CTC ACA GCG AGA AAC GAC TCG         144
Lys Glu Ile Ile Lys Thr Leu Asn Ile Leu Thr Ala Arg Asn Asp Ser
            35                  40                  45

TGC ATG GAG CTG ACC GTC ATG GAC GTC TTG GCA GCC CCT AAG AAC ACA         192
Cys Met Glu Leu Thr Val Met Asp Val Leu Ala Ala Pro Lys Asn Thr
        50                  55                  60

AGT GAC AAG GAA ATC TTC TGC AGA GCC ACA ACC GTG CTC CGG CAG ATC         240
Ser Asp Lys Glu Ile Phe Cys Arg Ala Thr Thr Val Leu Arg Gln Ile
    65                  70                  75

TAT ACA CAT CAC AAC TGC TCC ACC AAA TTC CTC AAA GGA CTC GAC AGG         288
Tyr Thr His His Asn Cys Ser Thr Lys Phe Leu Lys Gly Leu Asp Arg
 80                  85                  90                  95

AAC CTC AGC AGC ATG GCA AAC AGG ACC TGT TCT GTG AAT GAA GTC AAG         336
Asn Leu Ser Ser Met Ala Asn Arg Thr Cys Ser Val Asn Glu Val Lys
                100                 105                 110

AAG TGT ACA CTG AAA GAC TTC TTG GAA AGG CTA AAA GCG ATC ATG CAA         384
Lys Cys Thr Leu Lys Asp Phe Leu Glu Arg Leu Lys Ala Ile Met Gln
            115                 120                 125

AAG AAA TAC TCA AAG CAC TGAAGCTGAA TATCTTAATT TATGAGTTTT                432
Lys Lys Tyr Ser Lys His
        130

TTAATTACTT TATTTTAAAA ATATTTATAT ATTTATAATT CATTATAAAA TAAAGTATAT       492

GTAGAACCCG AAAAAAAAAA AAAAAAA                                           519

(2) INFORMATION FOR SEQ ID NO: 2:

(i) SEQUENCE CHARACTERISTICS:
(A) LENGTH: 133 amino acids
(B) TYPE: amino acid
(D) TOPOLOGY: linear

(ii) MOLECULE TYPE: protein

(xi) SEQUENCE DESCRIPTION: SEQ ID NO: 2:

Met Gly Leu Thr Tyr Gln Leu Ile Pro Ala Leu Val Cys Leu Leu Ala
1               5                   10                  15

Phe Thr Ser Thr Phe Val His Gly Gln Asn Phe Asn Asn Thr Leu Lys
            20                  25                  30

Glu Ile Ile Lys Thr Leu Asn Ile Leu Thr Ala Arg Asn Asp Ser Cys
        35                  40                  45

Met Glu Leu Thr Val Met Asp Val Leu Ala Ala Pro Lys Asn Thr Ser
    50                  55                  60

Asp Lys Glu Ile Phe Cys Arg Ala Thr Thr Val Leu Arg Gln Ile Tyr
65                  70                  75                  80

Thr His His Asn Cys Ser Thr Lys Phe Leu Lys Gly Leu Asp Arg Asn
                85                  90                  95

Leu Ser Ser Met Ala Asn Arg Thr Cys Ser Val Asn Glu Val Lys Lys
            100                 105                 110

Cys Thr Leu Lys Asp Phe Leu Glu Arg Leu Lys Ala Ile Met Gln Lys
        115                 120                 125

Lys Tyr Ser Lys His
    130

(2) INFORMATION FOR SEQ ID NO: 3:

    (i) SEQUENCE CHARACTERISTICS:
        (A) LENGTH: 133 amino acids
        (B) TYPE: amino acid
        (C) STRANDEDNESS: double
        (D) TOPOLOGY: linear

    (ii) MOLECULE TYPE: cDNA

    (iii) HYPOTHETICAL: NO

    (iv) ANTI-SENSE: NO

    (xi) SEQUENCE DESCRIPTION: SEQ ID NO: 3:

    Met Gly Leu Thr Ser Gln Leu Ile Pro Thr Leu Val Cys Leu Leu Ala
    1               5                   10                  15

    Cys Thr Ser Asn Phe Val His Gly His Lys Cys Asp Ile Thr Leu Gln
                20                  25                  30

Glu Ile Ile Lys Thr Leu Asn Ile Leu Thr Ala Arg Lys Asn Ser Cys
        35                  40                  45

Met Glu Leu Pro Gly Asp Asp Val Phe Ala Ala Pro Glu Asn Thr Arg
    50                  55                  60

Glu Lys Glu Thr Phe Cys Arg Ala Ser Thr Val Leu Arg His Ile Tyr
65                  70                  75                  80

Arg His His Thr Cys Met Lys Ser Leu Leu Ser Gly Leu Asp Arg Asn
                85                  90                  95

Leu Ser Ser Met Ala Asn Met Thr Cys Ser Val His Glu Ala Lys Lys
            100                 105                 110

Ser Thr Leu Lys Asp Phe Leu Glu Arg Leu Lys Thr Ile Met Lys Glu
        115                 120                 125

Lys Tyr Ser Lys Cys
    130

(2) INFORMATION FOR SEQ ID NO: 4:

     (i) SEQUENCE CHARACTERISTICS:
          (A) LENGTH: 135 amino acids
          (B) TYPE: amino acid
          (C) STRANDEDNESS: double
          (D) TOPOLOGY: linear

    (ii) MOLECULE TYPE: cDNA

   (iii) HYPOTHETICAL: NO

    (iv) ANTI-SENSE: NO

    (xi) SEQUENCE DESCRIPTION: SEQ ID NO: 4:

Met Gly Leu Thr Tyr Gln Leu Ile Pro Val Leu Val Cys Leu Leu Val
1               5                   10                  15

Cys Thr Ser His Phe Val His Gly His Lys Cys Asp Ile Thr Leu Ala
            20                  25                  30

Glu Ile Ile Lys Thr Leu Asn Ile Leu Thr Thr Arg Lys Asn Ser Cys
        35                  40                  45

Met Glu Leu Pro Val Ala Asp Val Phe Ala Ala Pro Lys Asn Thr Thr
    50                  55                  60

Glu Lys Glu Thr Phe Cys Arg Val Gly Ile Glu Leu Arg Arg Ile Tyr
65                  70                  75                  80

Arg Ser His Thr Cys Leu Asn Lys Phe Leu Gly Gly Leu Asp Arg Asn
                85                  90                  95

Leu Asn Ser Leu Ala Ser Lys Thr Cys Ser Val Asn Glu Ala Lys Thr
            100                 105                 110

Ser Thr Ser Thr Leu Lys Asp Leu Leu Glu Arg Leu Lys Thr Ile Met
        115                 120                 125

Lys Glu Lys Tyr Ser Lys Cys
    130                 135

(2) INFORMATION FOR SEQ ID NO: 5:

(i) SEQUENCE CHARACTERISTICS:
(A) LENGTH: 135 amino acids
(B) TYPE: amino acid
(C) STRANDEDNESS: double
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO: 5:

Met Gly Leu Thr Ser Gln Leu Ile Pro Val Leu Val Cys Leu Leu Ala
1               5                   10                  15

Cys Thr Ser His Phe Val His Gly His Lys Cys Asp Ile Thr Leu Glu
            20                  25                  30

Glu Ile Ile Lys Thr Leu Asn Ile Leu Thr Ala Arg Lys Asn Ser Cys
        35                  40                  45

Met Glu Leu Pro Val Ala Asp Val Phe Ala Ala Pro Lys Asn Thr Thr
    50                  55                  60

Glu Lys Glu Thr Leu Cys Arg Ala Gly Ile Glu Leu Arg Arg Ile Tyr
65                  70                  75                  80

Arg Ser His Thr Cys Leu Asn Arg Phe Leu Ser Arg Leu Asp Arg Asn
                85                  90                  95

Leu Ser Gly Leu Ala Ser Lys Thr Cys Ser Val Asn Glu Ala Lys Thr
            100                 105                 110

Ser Thr Ser Thr Leu Lys Asn Leu Leu Glu Arg Leu Lys Thr Ile Met
        115                 120                 125

Lys Glu Lys Tyr Ser Lys Cys
    130                 135

(2) INFORMATION FOR SEQ ID NO: 6:

     (i) SEQUENCE CHARACTERISTICS:
          (A) LENGTH: 153 amino acids
          (B) TYPE: amino acid
          (C) STRANDEDNESS: double
          (D) TOPOLOGY: linear

    (ii) MOLECULE TYPE: cDNA

   (iii) HYPOTHETICAL: NO

    (iv) ANTI-SENSE: NO

    (xi) SEQUENCE DESCRIPTION: SEQ ID NO: 6:

(2) INFORMATION FOR SEQ ID NO: 7:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(iv) ANTI-SENSE: NO

(xi) SEQUENCE DESCRIPTION: SEQ ID NO: 7:

GACTCGAGTC GACATCG                                                17

(2) INFORMATION FOR SEQ ID NO: 8:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(iv) ANTI-SENSE: NO

(xi) SEQUENCE DESCRIPTION: SEQ ID NO: 8:

GACTCGAGTC GACATCGTTT TTTTTTTTTT TTTT                             34

(2) INFORMATION FOR SEQ ID NO: 9:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(iv) ANTI-SENSE: NO

(xi) SEQUENCE DESCRIPTION: SEQ ID NO: 9:

TATTAATGGG TCTCACCTAC CA 22

## Description

### FIELD OF THE INVENTION

The present invention relates to feline Interleukin-4 corresponding to that found in feline peripheral blood lymphocytes, T cells, mast cells or tissues, and to novel forms and compositions thereof and particularly to the means and methods for its production to homogeneity in significant quantities.

The present invention further relates to the discovery of the DNA sequence and deduced amino acid sequence of feline Interleukin-4 as well as variants thereof. This discovery enabled the production of feline Interleukin-4 via the application of recombinant DNA technology, in turn, enabling the production of sufficient quality and quantity of material. Once recombinantly produced, the feline Interleukin-4 is utilized for therapeutical purposes for treatment of a variety of diseases and as immunological adjuvant. Monoclonal and polyclonal antibodies are also further embodiments of the present invention and can be utilized for the detection of natural feline Interleukin-4.

### BACKGROUND OF THE INVENTION

Cytokines, by definition, are soluble molecules produced by cells that mediate reactions between cells and generally act as hormones of the immune system. Lymphokines, categorized under the general definition of cytokines, are extracellular molecules generally produced by T lymphocytes that are associated with reactions of cell-mediated and humoral immunity.

Among the typical lymphokines are the Interleukins which are a group of antigen non-specific factors involved in lymphocyte activation and differentiation. Known Interleukins include Interleukin-1 (IL-1), Interleukin-2 (IL-2), Interleukin-3 (IL-3), Interleukin-4 (IL-4) and Interleukin-5 (IL-5), Interleukin-6 (IL-6), Interleukin-7 (IL-7), Interleukin-8 (IL-8), Interleukin-9 (IL-9), Interleukin-10 (IL-10), Interleukin-12 (IL-12), Interleuki-13 (IL-13) and Interleukin-15 (IL-15).

Interleukin-1 is produced by macrophages and other antigen producing cells. It acts on T cells to induce IL-2 and receptors. Interleukin-2 is produced by activated T cells and is necessary for the long term proliferation of T cells and can also act on previously stimulated B cells. Interleukin-3 is one of the colony stimulating factors produced by cell lines of several lineages which induces proliferation of lymphocytes. Interleukin-5 appears to play on important role in allergic and antiparasitic inflammatory responses.

In the class of Interleukins, Interleukin-4 represents a typical immunoregulatory lymphokine. See, for example Finkelman et al, Ann. Rev. Immuno.,8, 303-333 (1990); and Paul Blood, 77, 1859-1870 (1991). It is produced mainly by activated T cells and mast cells and influences various cell types of the lymphoïd system. Interleukin-4 is a key regulatory molecule in T helper cell type-2 (hereinafter referred to as Th-2) driven immune responses that are considered necessary for the elimination of extracellular pathogens. (Scott and Kaufman, Immunol. Today 12 : 346-348 (1991). B cells are stimulated by Interleukin-4 *in vitro* to express class II major histocompatibility complex molecules, the IgE low affinity receptor (CD23) and immunoglobulins class E and G1. DNA synthesis is induced in activated B cells, mature T cells, foetal thymocytes, mast cells and others.

Mice treated with neutralizing antibodies directed against Interleukin-4 or against the Interleukin-4 receptor ( Finkelman et al, *supra*; Urban, Jr et al, P.N.A.S., USA 88, 5513-5517 (1991) ) are unable to produce IgE and show reduced serum levels of IgGI after challenge with relevant stimuli. This indicates a pathophysiological role in the generation of disease states such as hyper-IgE syndrome or IgE-mediated allergic conditions (Finkelman et al, supra, Tepper et al, Cell, 62 457-467,(1990). Furthermore, Interleukin-4 deficient mice have strongly reduced Th2 cytokine responses (Kopf et al, Nature, 362, 245-247 (1993) which suggests that-Interleukin-4 is required for the generation of the Th-2 derived cytokines and that immune responses dependent on these cytokines are impaired.

Due to its inhibitory effects on Interferon Gamma (IFN γ), Interleukin-4 is considered to down-regulate T helper cell type-1 (Th-1) associated immune responses that are necessary for the control of many intracellular pathogens. Recently, it has been shown that neutralization of Interleukin-4 at the time of immunization with inactivated respiratory syncytial virus improves T helper cell type-1 immune responses and reduces illness against challenge infection (Tang and Graham, J.Clin. Invest., 94:953-1958 (1994). Furthermore, the induction of down-regulator cytokines may be an important factor limiting the efficacy of certain vaccination protocols.

Human and murine Interleukin-4 have already been cloned (Yokota, T. et al, PNAS 83:5894-5898 (1986) [human] ; Lee, F. et al, PNAS 83:2061-2065 (1986) [mouse]). The nucleotide sequences from the murine and human cDNA clones showed an open reading frame coding for approximately 140 amino acids, the first 20 of which are hydrophobic and indicative of the signal peptide sequences usually associated with secreted proteins Paul et al Ann. Rev Immunol, 5: 429 (1987) and Yokota et al supra. The human and murine clones share 50% homology at the amino acids level but there is no species cross-reactivity.

Although the murine and human Interleukin-4 nucleotide sequences have already been identified, nothing is known about feline Interleukin-4. Indeed, wild feline species and especially cats suffer a variety of diseases in which Interleukin-4 probably plays a role. These diseases include, but are not limited to, allergies, feline infectious peritonitis virus (FIPV), feline immunodeficiency virus (FIV), feline leukemia virus (FeLV) as well as autoimmune diseases.

Moreover, Interleukin-4 may also be used as a prophylactical immunomodulator during vaccination of Felidae against microbial and viral pathogens, tumors and metastasis or as a therapeutic immunomodulator in infectious diseases and tumors.

Accordingly, it is an object of this invention to characterize feline Interleukin-4 and to clone feline Interleukin-4 such that this lymphokine can be produced in large amounts. In yet another aspect, the present invention provides mutant variants of single amino acid substitutions and other variant mutant sequences with amino acid subsitutions at the C-terminal end which are generated to antagonize the function of feline Interleukin-4. In yet another aspect, the present invention provides antibodies such as monoclonal and polyclonal antibodies directed against Interleukin-4. In addition, veterinary compositions are provided utilizing essentially purified Interleukin-4 derived from the expressed polypeptide.

These and other objects are achieved by the present invention as evidenced by the summary of the invention, description of the preferred embodiments and the claims.

### SUMMARY OF THE INVENTION

The present invention provides the complete nucleotide sequence of feline Interleukin-4 and the deduced amino acid sequence.

In one of its composition aspects, the present invention is directed to a novel feline cDNA obtained by gene cloning and nucleotide sequencing.

Yet another composition aspect of the present invention is directed to polypeptides of feline Interleukin-4 invention and provides a process for producing essentially purified feline Interleukin-4.

Yet another composition aspect of the present invention is directed to single amino acid substitutions of the Interleukin-4 sequence that results in antagonists with high receptor binding activity but decreased signal generation.

Another aspect of the present invention provides monoclonal and polyclonal antibodies directed against feline Interleukin-4.

In yet another aspect, the present invention provides nucleic acid probes for detection of Interleukin-4.

In yet another aspect, the present invention provides veterinary compositions comprising essentially pure Interleukin-4.

In yet another aspect, the present invention provides the use of cDNA feline Interleukin-4 sequence for naked DNA vaccines.

### BRIEF DESCRIPTION OF THE DRAWINGS

Fig.1 is the nucleotide sequence and deduced amino acid sequence of Interleukin-4 including the precursor peptide, (SEQ.I.D. No.1).

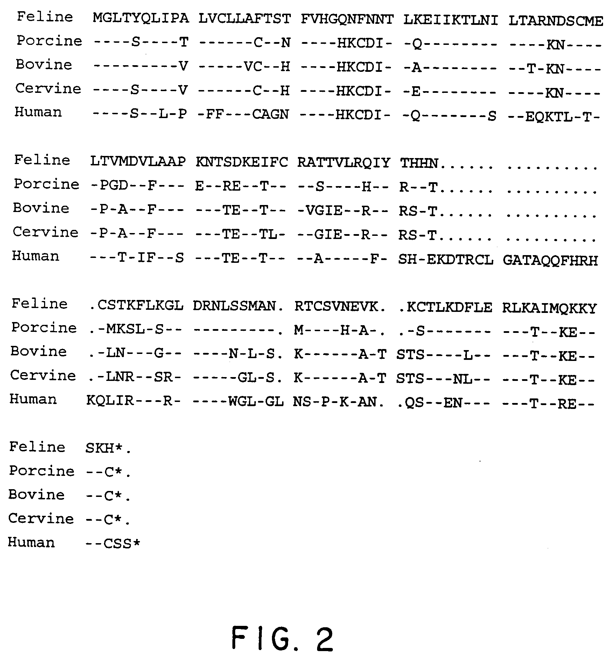

Fig.2 is an alignment of amino acid sequences of feline, porcine, bovine, cervine and human Interleukin-4, (SEQ.I.D. Nos.1 to 5). A dash indicates homology with the feline sequence. A point indicates absence of a corresponding amino acid.

### DETAILED DESCRIPTION OF THE PREFERRED EMBODIMENTS OF THE INVENTION

As used herein, the term "feline" encompasses any member of the family Felidae including the common domestic cat, as well as wild feline species such as the tiger, lion, leopard, mountain lion, lynx, bobcat, ocelot and the like.

The term "essentially pure form" when used to describe the state of feline Interleukin-4 produced by the invention means free of protein or other materials normally associated with feline Interleukin-4 when produced by non-recombinant cells; i.e., in its "native environment".

The term "mutant variants" encompasses at least one, preferably one to ten, more preferably one to twenty, most preferably one to thirty amino acid substitutions in the feline Interleukin-4 molecule.

As used herein, the term "fragment" encompasses part of the amino acid sequence or nucleotide sequence of Interleukin-4. When referring to an "amino acid fragment" generally about 1 to about 50, preferably about 1 to about 25, most preferably about 1 to about 10 amino acids of the entire Interleukin-4 sequence is contemplated. When referring to the term nucleotide fragment, generally about 1 to about 100, preferably about 1 to 50, most preferably about 1 to about 25 nucleotides of the Interleukin-4 sequence is contemplated.

More specifically, the present invention relates to the molecular cloning of feline Interleukin-4 and the sequencing of the cloned inserts containing Interleukin-4. Once the Interleukin-4 sequence is obtained, it is then expressed via expression vectors and the Interleukin-4 is recovered from the culture media. Furthermore, monoclonal and polyclonal antibodies are then generated following known procedures in the art.

Although it is known that Interleukin-4 derived from various species exhibits no species cross-reactivity such as in humans, murine, bovine and porcine, the phylogenetic relationship between cats and dogs suggests that there may be some cross-reactivity between feline and dog Interleukin-4 molecules. Therefore, although the present invention details the molecular cloning from the feline species, the procedures and methods described herein as well as the compositions equally apply to dogs.

Very generally, the procedure utilized for cloning the feline Interleukin-4 encompasses the isolation of crude preparations of Interleukin-4 from various cells and tissues known to contain this cytokine. Examples include, but are not limited to, mononuclear lymphocytes from lymphoïd tissues such as spleen tissue, lymph glands, subepithileal tissue such as tonsils, adenoids and Peyers patches and the like. In addition mononuclear lymphocytes can also be isolated from blood of cats.

Besides using mononuclear lymphocytes, mast cells and T cells can also be used as a source to obtain Interleukin-4 for cloning purposes. However it is preferably to utilize as a starting material lymphoïd tissue, most preferably tissue derived from the spleen of a cat or feline blood.

The tissues or blood is then extracted from the cat by methods known in the art. In the case of using tissue, for example from spleen, the tissue is excised, minced, and pressed through a filter (NPBI, EMMER-COMPASCUM, The Netherlands) in a lymphocyte medium. In the case of blood, the sample is withdrawn via a syringe from the cat into a tube containing an anitcoagulent such as EDTA, heparin ACD and the like. Defribrinated blood can also be used.

The isolated splenocytes or peripheral lymphocytes from blood are then diluted by addition of an equal amount of about 0.9% NaCl. About 6 ml of this diluted preparation is then layered over about 3 ml of Lymphoprep™ (NYCOMED PHARMA AS, Norway) in a 12 to 15 mm centrifuge tube. Alternatively, Lymphoprep™ can be underlayered.

The centrifuge tubes are then capped and centrifuged at 800 x g for about 15 to 30 minutes, preferably 20 minutes at room temperature in a swing-out rotor. After centrifugation, the mononuclear cells form a distinct band at the sample/medium interface. The cells are then removed from this interface, using, for example, a Pasteur pipette.

The harvested cells can be further diluted with 0.9% NaCl or a lymphocyte medium to reduce the density of the solution and further pelleted by centrifugation from 5 to 20 minutes at about 250 X g.

The isolated cells are cultured in a lymphocyte medium (Dubbecco's minimal essential medium, DMEM, containing 10% fetal calf serum, penicillin and streptomycin) containing 5 µg/ml of concavalin-A, at a concentration of 10⁶ cells/ml.

The pelleted lymphocytes are further subjected to disruption using a detergent. Any detergent can be utilized in this step such as Triton-X-100, cholic acid, deoxycholic acid, lauryl sulfate and the like.

After disrupting the cells, total RNA is then isolated using any general procedure described in the art. such as proteinase K lysation, followed by phenol:chloroform extraction, guanidium triocyanate extraction, followed by cesium chloride gradients, guanidine hydrochloride and organic solvent extraction, the guanidium isothiocyanade method and the like. It is preferable to use the method described by Boom et al, Journal of Clinical Micro., 28, 495-503 (1990).

Basically, the Boom et al procedure involves placing the lymphocyte suspension into test tubes containing a solid carrier such as size-fractionated silica particles or diatoms and a guanidiinium thiocyanate containing lysis buffer (120 g guanidinium thiocynate in 100 ml 0.1 M Tris hydrochloride, pH 6.4; 22 ml of 0.2 M EDTA solution (Merck) adjusted to pH 8.0 and 2.6 g of TritonX-100™ were added and the solution was homogenized). The pelleted lymphocytes were then mixed with the other substances in test tubes and incubated for about 8 to 12 minutes at room temperature. After the incubation period, the test tubes were vortexed again and centrifuged for about 15 seconds in a fixed angle rotor at about 12,000 x g. The supernatent was then siphoned off and the remaining silica pellet containing the total RNA derived from the lymphocytes was washed twice with a buffer containing 120 g of guanidinium thiocynate in 100 ml of 0.1 M Tris hydrochloride, pH 6.4, twice with 70% (v/v) ethanol, and once with acetone. After siphoning off the acetone, the reaction tubes were dried at about 56° C for about 5 to 15 minutes.

To elute the total RNA from the particles in the tubes the tubes are further vortexed briefly and centrifuged for about 1 to 3 minutes at 12,000 x g. The solution containing total RNA was then decanted.

The mRNA obtained is then reverse transcribed into cDNA using a 35-base oligonucleotide primer containing 17 dT residues and an adaptor sequence. This adaptor sequence was derived from an antisense adaptor primer, later utilized in the polymerase chain reaction (PCR) and has the following specific sequence:
5' GACTCGAGTCGACATCG 3'. (SEQ. I.D. No. 6)

Thus, the 35-base oligonucleotide primer used in the reverse transcriptase reaction is:
5' GACTCGAGTCGACATCGTTTTTTTTTTTTTTTTT 3' (SEQ I.D. No. 7).

The feline Interleukin-4 was further amplified by PCR using a sense primer based on the conserved regions of the human, porcine and bovine Interleukin-4 gene and the antisense primer described above as SEQ. I.D. No.6. This sense primer has the following sequence:
5' TATTAATGGGTCTCACCTACCA 3' (SEQ. I.D. No. 8)

The protocol used for the PCR reaction is described by Frohman et al in PNAS USA 85:8998-9002 (1988). This procedure is a simple and efficient cDNA cloning strategy termed "rapid amplification of cDNA ends" (RACE) that involves the use of the polymerase chain reaction to amplify copies of the region betweeen a single point in the transcript and the 3' or 5' end. The minimum information needed for the RACE procedure is a single short stretch of sequence within the mRNA to be cloned. The present invention is not limited to the RACE method. Indeed, any other method can be utilized to generate the cDNA including the synthetic synthesis thereof.

More specifically, the PCR amplification protocol which is utilized is the following. About 0.5 µl to 5 µl cDNA, between about 20 to 30 pmol of the adaptor primer (SeQ.I.D.No:6), between about 30 to 60 µl of PCR cocktail containing about 10%-20% (vol/vol) dimethyl sulfoxide (DmSO)/1 x *Taq* polymerase buffer (New England Biolabs)/ and about 1.5 mM of each dNTP were denatured for about 5 minutes at about 90°-100°C and cooled to about 65°-75°C. Approximately about 2.0 to 4.5 units of *Taq* DNA polymerase (Perkin-Elmer-Cetus) was added and the mixture was overlaid with about 20 µl to 40 µl of mineral oil (Sigma) at 70°-75°C and annealed between 50°-58°C for approximately 2 minutes. The cDNA was extended at 70°-75°C for 35-60 minutes. Using a DNA Thermal Cycler (Perkin-Elmer-Cetus) about 30 to 60 cycles of amplification is carried out using a step program of 90°-95°C, 40sec; 50°-58°C, 2 minutes; 70°-75°C, 3 minutes, followed by a 10-20 minutes final extension at about 65°-75°C.

After generating the appropriate amounts of cDNA, the amplified fragment is ligated into a plasmid and transfected in *E.* coli. The transformed clones were sequenced by dideoxy sequencing as described by Sanger et al., PNAS,74 5463 (1977). Any plasmid can be utilized for the sequencing of the cDNA including, but not limited to pSP64, pSP65, pGEM-3, pGEM-3z, pGEM-3zf(-), pGEM-T, pGEM-47 and the like. It is preferable to use pGEM-T.

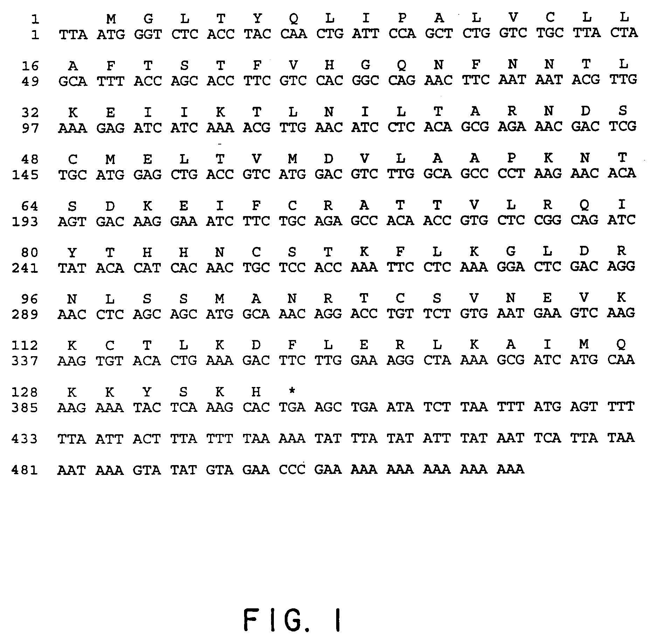

The 522 base pair feline Interleukin-4 cDNA sequence contains an open reading frame encoding a 133 amino acid protein as illustrated in Figure 1 (SEQ I.D. NO: 1). Cleavage of the precursor polypeptide into the mature peptide, illustrated in Figure 1 (SEQ I.D. No. 1), probably occurs after the Glycine 24 residue. A comparison of the predicted amino acid sequence revealed that feline Interleukin-4 shares 72%, 67%, 66% and 50% homology with porcine, bovine, cervine and human Interleukin-4, respectively.

The feline Interleukin-4 cDNA containing plasmid pNoTA/T7 in competent *E. coli* cells was deposited on August 8, 1995 at the Centraalbureau Voor Schimmelcultures (CBS), Oosterstraat 1, Postbus 273, NL-3740 AG Baarn, the Netherlands and was assigned accession n° CBS 614.95.

Once the sequence was determined, the entire cDNA was cloned into an expression vector and Interleukin-4 was expressed. Any expression vector can be used such as plasmid vectors for expression in *E. coli* such as pBR322, pUC18, pUC19, pUC118, pUC199 and the like. Bacteriophage vectors are also contemplated for expression, also. Mammalian expression vectors such as pJB8, c2RB, pcos1EMBL, pEW15, pEW16, SV40 and the like can be also used to express feline Interleukin-4. All of these expression vectors are well known in the art and described in Sambrook et al *supra*. It is preferable to use pAcDZ1 for transfection and recombination with nuclear polyhedrosis virus in insect cells.

More specifically, the PCR amplified fragments containing the cDNA of Interleukin-4 were ligated into pNoTA/T7 using a "Primer PCR cloner kit" from "5 prime to 3 prime" (Boulder, Colorado). The fragments were then digested with *Bam*HI and subcloned into pAcDZ1. After transfection-infection, the recombinant baculoviruses were derived from nuclear polyhedrosis virus and grown at 28°C in Spodoptera frugiperda (Sf21) cells in TC-100 insect-cell medium.

Once expressed, the Interleukin-4 is further purified from the culture media. The purified Interleukin-4 can then be injected into mice to produce monoclonal antibodies according to the procedure of Kohler and Milstein et al Nature, 256:495 (1975). Polyclonal antibodies can also be prepared according to the methods described in Sambrook et al *supra*.

These monoclonal and polyclonal antibodies are used to detect natural feline Interleukin-4 in radioimmunosassys, ELISA's (enzyme-linked immunosorbent assay) and the like. These assays are used to identify and detect type-2 immune responses in felines and dogs which are characterized by high Interleukin-4 production and by high antibody responses that, for example are indicative of allergies. By high Interleukin-4 production is meant that Interleukin-4 has levels above the normal control in these assays.

Mutant variants of the feline sequence for Interleukin-4 are useful as prophylactical immunomodulators during vaccination of *Felidae* against microbial and viral pathogenes, tumors and metastasis, Interleukin-4 can also be used as a therapeutic immonomodulator in infectious diseases and tumors.

These mutant variants can be obtained by the alteration of a single amino acid in the C-terminal end of the feline Interleukin-4 amino acid sequence using, for example, site-directed mutagenesis as described by Heffron et al., P.N.A.S., 78: 6012 (1978); Melgar and Goldthwait, J. Biol. Chem, 245 (1968); and Sambrook et al *supra*. For example a single point mutation of the amino acid at position 106 in SEQ. I.D. No. 1 wherein the amino acid Y (tyrosine) has been changed to D (aspartic acid) provides a mutant variant of the feline Interleukin-4 sequence that likely acts as an antagonist that binds to cell receptors but fails to mediate signal transduction. Additional mutant variants that are preferred embodiments of the present invention include but are not limited to altering each of the 20 amino acids of the C-terminal end of feline Interleukin-4.

Moreover, random mutagenesis of the C-terminal end of Interleukin-4 using the procedure described by Lehtovaara et al., Protein Engineering, vol. 2 no. 1:63-68 (1988) is another preferred embodiment that provides mutant variants of feline Interleukin-4 that can be used as Interleukin-4 antagonists as well.

Labeled nucleotide probes derived from sections of the feline Interleukin-4 sequence such as those from nucleotide position 1 to nucleotide position 50, nucleotide at position 51 to nucleotide at position 68, nulceotide position 69 to nucleotide position 100, nucleotide position 101 to nucleotide position 130, nucleotide position 131 to nucleotide position 168 of SEQ I.D. No. 1 and the like are yet another preferred embodiment of the present invention. The appropriate nucleotide sequences are labeled by methods known in the art such as with radioactive labels, enzymes and the like which are described in Sambrook et al supra. These probes can be utilized to detect Interleukin-4-mRNA sequences in felines, as well as in related sequences.

Another preferred embodiment of the present invention is the use of a fragment of the DNA sequence of SEQ. I.D. No. 1 as a primer set. More specifically, this primer set encompasses from 15 to 20 nucleotides and may include nucleotides at positions 1 to 10 or 10 to 20 or 20 to 30 or 30 to 40 or 50 to 60 etc. up to and including nucleotide at position number 522 of SEQ. I.D. No:1 in increments of either 5 to 10 successive nucleotide sequences.

Yet another preferred embodiment of the present invention includes feline Interleukin-4 specific primers or probes that encompass an intron, thus allowing the differentiation between PCR products of genomic DNA versus mRNA-derived cDNA, can be used to identify Interleukin-4 gene transcripts from *ex vivo* stimulated or unstimulated cells.

A veterinary composition is also another aspect of the present invention, said veterinary composition comprising an effective amount of Interleukin-4 in any pharmaceutically effective carrier vehicle. Among the pharmaceutically effective carriers are contemplated, but limited to saline, serum albumin and the like. Suitable vehicles and their formulation are described in Remington's, *Pharmaceutical Sciences* by E.W. Martin, which is hereby incorporated herein by reference.

Naked DNA vaccines are also contemplated for treating tumors in cats and dogs. Such naked DNA vaccines are described in Biofutur May 1995. The expressed Interleukin-4 in plasmid form is diluted in saline and injected directly subcutaneously, systematically or into a muscle of a cat. Between about 10 µl to 80 µl of Interleukin-4 is diluted in about 3 ml of saline. The resulting mixture is vortexed, placed in a syringe and administered to the cat for tumor treatment.

In order to further illustrate the present invention and advatages thereof, the following specific examples are given, it being understood that the same are intended only as illustrative and in nowise limited.

### Example 1

### Isolation of Mononuclear Lymphocytes From Feline Blood

Cat's blood was drawn using a syringe into a test tube containing EDTA. Approximately 5 ml was obatined. The blood was diluted with 5 ml of 0.9% NaCl. 6 ml of the diluted blood was then layered over 3 ml of Lymphoprep™ in a 15 mm test tube. The tube was capped and centrigfuged at 800 x g for 20 minutes at room temprature in a swing-out rotor. After centrifugation, the mononuclear lymphocytes formed a distinct band at the sample-medium interface. This band was then removed with a Pasteur pipette without removing the upper layer. The mononuclear lymphocytes were then diluted with 0.9% NaCl to reduce the density of the solution and the cells were pelletted by centrifugation at 350 x g for 10 minutes. The cells were washed in lymphocyte medium and cultured at 10 ⁶ cells/ml. The cells were stimulated with concanavalin-A (5 µg/ml), for 5 hours.

### Example 2

### Isolation of mRNA

mRNA was isolated using the procedure described by Boom et al *supra*.

### Preparation of size-fractionated silica

Silica particles (60 g of silicon dioxide, SIGMA CHEMICAL CO.) were suspended in demineralized water in a total volume of 500 ml in a glass cylinder and sedimented at unit gravity for 24 hrs. at room temperature. A 430 ml portion of the supernatant was then disposed of by suction, demineralized water was added to a total volume of 500 ml and the silica pellet was suspended by further shaking. After another sedimentation step for 5 hrs at room temperature, 440 ml of supernatant was disposed of by suction and 600 µl of HCL (32% wt./vol) was added to adjust the suspension to pH2. The resulting suspension was further autoclaved for 20 minutes at 121°C and sheared in 4 ml portions at room temperature.

### Preparation of Reaction Vessels

Reaction vessels were prepared by adding 900 µl of lysis buffer which was made by dissolving 120g of guanidinium thiocynate in 100 ml of Tris hydrochloride, pH 6.4 and subsequently adding 22 ml of 0.2 M EDTA adjusted to pH 8.0 and 2.6 g of TritonX-100™. The solution was homogenized. 40 µl of the prepared silica particles were then added to a 1.5 ml Eppendorf reaction tube and the solution was mixed by vortexing. 50 µl of the mononuclear lymphocytes was then added and the vessel was vortexed for about 5 seconds. The reaction vessel was then incubated for 10 minutes at room temperature, vortexed once again for 5 seconds and centrifuged for 15 seconds in an Eppendorf microfuge at 12,000 x g. The supernatant was disposed of by suction and the silica pellet was washed twice with a buffer containing 120 g of guanidinium thiocynate in 100 ml of 0.1 M Tris hydrochloride, pH 6.4, twice with 70% (v/v) ethanol, and once with acetone. After siphoning off the acetone, the reaction tubes were dried at about 56° C for 10 minutes. The vessel was briefly vortexed again and centrifuged for 2 minutes at 12,000 x g. The solution containing total RNA was then decanted.

### Example 3

### Reverse Transcription of mRNA into cDNA

One microgram of RNA was dissolved in 16.5 µl of distilled water and was heated to 65°C for 3 min, quenched on ice, added to 2 µl of 10 X RTC buffer (1 X RTC buffer is 50 mM Tris-HCl, pH 8.15 at 41°C/6mM MgCl₂/40 mM KCl/1 mM dithiothreitol/each dNTP at 1.5 mM) 0.25 µl (10 units) of RNasin (Promega Biotec, Madison,WI), 0.5 µl of (dT) 17 adaptor (1 µg/µl), 5' GACTCGAGTCGACATCGTTTTTTTTTTTTTTTTT 3' (SEQ I.D. No. 4). and 10 units of avian myeloblastosis virus reverse transcriptase (Life Sciences, Saint Petesburg, FL), and incubated for 2 hr at 41°C. The reaction mixture was diluted to 1 ml with TE (10 mM Tris-HCl, pH 7.5/l mM EDTA) and stored at 4°C.

*Amplification*. The cDNA pool (1 µl) and amplification (3' amp) and adaptor primers of 5' GACTCGAGTCGACATCG 3' (SEQ. I.D. No. 4) and 5' TATTTAATGGGTCTCACCTACCA 3' (SEQ. ID No. 6 ) (25 pmol each) in 50 µl of PCR cocktail [10 % (vol/vol) dimethyl sulfoxide/1 X *Taq* polymerase buffer (New England Biolabs/each dNTP at 1.5 mM] were denatured (5 min, 95°C) and cooled to 72°C. Then 2.5 units of *Thermus aquaticus (Taq)* DNA polymerase (Perkin-Elmer-Cetus) was added and the mixture was overlaid with 30 µl of mineral oil (Sigma) at 72°C and annealed at 50°-58°C for 2 min. The cDNA was extended at 72°C for 40 min. using a DNA Thermal Cycler (Perkin-Elmer-Cetus), 40 cycles of amplification was carried out using a step program (94°C, 40 sec ; 50°-58°C, 2 min ; 72°C, 3 min), followed by a 15-min final extension at 72°C.

### Example 4

### Sequencing of Interleukin-4

After generating the appropriate amounts of cDNA, the amplified fragment was ligated into pGEM-T and transfected in *E.* coli. The transformed clones were sequenced by dideoxy sequencing as described by Sanger et al., PNAS,74 5463 (1977). The cDNA and deduced amino acid sequence is set forth in Figure 1.

### Example 5

### Expression of feline Interleukin-4

The PCR fragments containing the cDNA of Interleukin-4 were ligated into a pNOTA/T7, using the "Prime PCR cloner kit" from 5 prime to 3 prime INC, Boulder, Colorado and subcloned after *Bam*HI digestion into pAcDZ1. This vector contains recombinant sequences for homologous recombination in wild-type baculovirus. The recombinant baculoviruses were derived from nuclear polyhedrosis viruses and grown at 28°C in "Spodoptera frugiperda" (Sf21) cells in TC-100 media. The expressed Interleukin-4 was further purified.

### Example 6

### Preparation of Antibodies

30 µg of expressed feline Interleukin-4 is dissolved in 100 µl Tris-HCl buffer and emulsified with 130 µl of Freund's complete adjuvant. 50 µl of the emulsified antigen-adjuvant is injected subcutaneously at four sites in mice. The mice are then boosted with an interperitoneal injection of 100 µl of Tris-HCl buffer containing 8 µl of soluble antigen. The mice are then bled from the tail vein 7 to 10 days after the booster injection and are tested for the presence of Interleukin-4 antibody.

Monoclonal antibodies are prepared by the method of Kohler and Milstein, *supra*. These anitbodies are then further used in an ELISA assay to detect Interleukin-4 levels in felines.

### Example 7

### Naked Vaccines

30 µl of the expressed Interleukin-4 from Example 5 is diluted in saline and administered (several times) systemically to a cat that has a tumor or directly into the visible tumor. It is shown that this injection reduced the size of cat's tumor.

### Example 8

The feline Interleukin-4 cDNA can be transferred either in isolated tumor cells or directly into the tumor or systemically to induce tumor rejection and immunity to metastasis. Direct injection of feline Interleukin-4 cDNA can be used to stimulate and direct immune response during vaccinations or as therapy against tumors, infections disease, autoimmune diseases, etc.

### Example 9

### Nucleotide Probes

The nucleotide sequence of nucleotides 145 to 150 (by way of example) are synthesized using an automated nucleotide synthesizer or by using the method described by Itakura et al Annu. Rev. Biochem. 53:323 (1984). The nucleotide sequence is then labeled with radioactive ³²P using the methods described by Sambrook et al *supra*. Under stringent conditions, the radiolableled probes are then used to test for the presence of Interleukin-4 in cats and dogs.

## Claims

1. Feline Interleukin-4.

2. The feline Interleukin-4 according to Claim 1, comprising the nucleotide sequence of SEQ. I.D. No.1 as shown in Figure 1.

3. The feline Interleukin-4 according to Claim 1, comprising the amino acid sequence of Sequence I.D. No 1 as shown in Figure 1.

4. Monoclonal antibodies directed against feline Interleukin-4.

5. A mutant variant of feline Interleukin-4 of Claim 3.

6. A veterinary composition comprising an effective amount of Interleukin 4 according to Claim 1 or Claim 5 in a pharmaceutically acceptable vehicle.

7. The mutant variant according to Claim 5, wherein the tyrosine at amino acid position 106 is changed to aspartic acid.

8. A method to synthesize large quantities of Interleukin-4 comprising the steps of:
a) expressing a DNA sequence encoding feline Interleukin-4 in a recombinant host cell with an expression vector containing said DNA sequence; and
b) recovering said expressed Interleukin-4.

9. The process according to Claim 8, wherein the recombination vector is pAcDZ1 for expression in recombinant polyhedrosis virus in insect cells.

10. The process according to Claim 9, wherein said recombinant host microorganism are insect cells.

11. The process according to Claim 10, wherein said insect cells are Spodoptera frugiperda cells.

12. A vaccine comprising the Interleukin-4 made by the process of Claim 6 or Claim 8.

13. A nucleotide sequence encoding the polypeptide of SEQ. I.D. No. 1 as set forth in Figure 1.

14. A nucleotide probe or specific primer sets comprising a fragment of the DNA sequence in SEQ. I.D. No. 1 as set forth in Figure 1 which is labeled with a radioactive label.

15. The mutant variant according to Claim 5, wherein said mutant variant comprises a single point mutation at one of the the last twenty amino acid positions of SEQ I.D. No. 1.

16. An antagonist that binds to cell receptors of feline Interleukin-4 but fails to mediate signal transduction said antagonist comprising the mutant variants of any one of Claims 5, 7 or 15.
